# EUROPEAN PATENT SPECIFICATION

(11) **EP 3 320 951 B1**
(45) Date of publication and mention of the grant of the patent: **30.12.2020**
(21) Application number: 17200978.9
(22) Date of filing: 10.11.2017
(51) Int. Cl.: A61N 1/40, A61N 7/00, A61N 5/06

(54) **APPARATUS FOR THERAPEUTIC AND/OR COSMETIC TREATMENTS**
GERÄT FÜR THERAPEUTISCHE UND / ODER KOSMETISCHE BEHANDLUNGEN
APPAREIL POUR TRAITEMENTS THÉRAPEUTIQUES ET / OU COSMÉTIQUES

(30) Priority: 11.11.2016 IT 201600113932
(43) Date of publication of application: 16.05.2018
(73) Proprietor: Winform Medical Engineering S.r.l., 30027 San Dona' di Piave (Venezia) (IT)
(72) Inventor: PIZZOCHERO, Franco, 30027 SAN DONA' DI PIAVE (VENEZIA) (IT)
(74) Representative: Piovesana, Paolo

(56) References cited:
- EP-A1- 2 614 807
- WO-A1-2007/093998
- WO-A1-2013/076714
- WO-A2-2014/138582
- DE-A1-102011 102 570
- US-A1- 2010 137 752
- US-A1- 2011 015 549

## Description

The present invention relates to an apparatus with an applicator handpiece for therapeutic and/or cosmetic treatments.

As is known, the dispensing of electric currents at high frequency on biological tissues causes an increase of temperature (diathermy) of the treated zones, and this is perceived as a sensation of heat in the deep and superficial tissues of the musculoskeletal apparatus. To date, diathermy, also known as CareTherapy® (which is the acronym for "capacitive and resistive energy transfer") allows to obtain a biostimulation capable of affecting, both at a superficial and deeper level, the soft tissues and those of tendon and bone.

It is also known that ultrasounds, which are mechanical sound waves characterized by frequencies comprised between 0.5 MHz and 15 MHz (i.e. greater than those of the band 16 Hz ÷ 20 kHz audible to the human ear), when they propagate through the biological tissues, cause both thermal effects and mechanical effects, such as cavitation.

In the medical field (for example in urology for the destruction of kidney stones or in the orthopedic/physiatric field for the generation of a therapeutic effect inside tissues such as bones, muscles, ligaments and tendons), it is known to use shock waves, i.e. mechanical waves with wave shapes characterized by a quick peak of positive pressure followed by a peak of negative pressure and capable of producing a direct mechanical stimulation. Among known equipment for the generation of shock waves, there are those which are based on the piezoelectric phenomenon since the very quick initial peak of pressure is generated subjecting one or more piezoelectric crystals to a suitable alternating current field.

Currently, there are several apparatuses with applicator handpieces, however they are not fully satisfactory since they allow the application of only one type of treatment at a time. In particular, in the case of treatment by diathermy the user must use one type of handpiece, while in the case of treatment by ultrasound and/or shock waves, the user must use a different type of handpiece. It is understood that this is not convenient since, in order to modify the type of treatment, the user must change the type of handpiece, with consequent increase of time and of industry of the required operations.

WO2013/076716 describes an applicator element to be positioned in contact with the skin and provided with electrodes, which provide a voltage in radio frequency such as to provide the heating of the skin in contact, and with transducers for the generation of ultrasounds.

EP2614807 describes equipment provided with an array of individually controlled units for skin treatment. In particular, each unit is provided with a cavity communicating with a vacuum source so as to suck the portion of the skin to be treated and thus create a protrusion. Furthermore, inside the inner walls of said cavity, electrodes for radio frequency and ultrasound transducers are provided.

DE102011102570 describes a handpieces provided with a shock wave and electrode generator for electro-stimulation (i.e. of alternating electric pulses at low frequency, i.e. at most 120 Hz). Furthermore, the handpiece comprises a support handle of an application head substantially cup-shaped.

US2010/0137752 describes equipment for the treatment by pneumatic shock waves, i.e. generated by the use of compressed air. In particular such equipment comprises a handpiece of tubular shape provided with an inlet for compressed air which moves, inside a guiding tube, a percussion element which, whenever it strikes the applicator element, causes the generation of a shock wave.

WO2007/093998 describes an apparatus for the treatment of adipose tissue wherein the applicator is provided with electrodes for radio frequency, to cause the controlled heating of the tissue, and with transducers to generate ultrasounds of extremely high intensity (greater than 4 MPa, i.e. 40 bars) at a frequency less than 1 MHz, preferably less than 300 kHz, and with a power density of about 700 W/cm². In particular, the head of the applicator has the traditional dome shape and is further provided with means - for example for suction by a vacuum source or by mutual approach of two plates applied to the skin to be treated - for creating a protrusion of said skin.

US2011/0015549 describes an apparatus which is specifically and only intended to treat nails. In particular, such an apparatus comprises an applicator which has a lower base to support the finger during the nail treatment of the same finger and an upper base provided with electrodes for radio frequency and with transducers for ultrasounds.

WO2014/138582 only describes an apparatus for the electro-hydraulic generation of shock waves.

It is the object of the invention to provide an apparatus with an Handpiece which overcomes the drawbacks of traditional devices and is improved and/or alternative with respect thereto.

It is another object of the invention to provide an apparatus with an applicator handpiece which also allows to carry out combined diathermy and shock wave treatments.

It is another object of the invention to provide an apparatus with an applicator handpiece which, during the single and/or combined treatment, allows and facilitates the transfer of a phytochemical useful for the treatment itself to the skin.

It is another object of the invention to provide an apparatus with an applicator handpiece which is simple, quick and intuitive to use.

It is another object of the invention to provide an apparatus with an applicator handpiece with an alternative feature, both in manufacturing and operating terms, with respect to the traditional ones.

It is another object of the invention to provide an apparatus with an applicator handpiece which allows to simultaneously carry out multiple different treatments so as to suitably and synergistically combine the beneficial effect of each of them.

It is another object of the invention to provide an apparatus with an applicator handpiece which provides the user with a mean of action to intervene, simultaneously on the same subject or sequentially on different subjects, on more diseases validated in the field of treatment by diathermy, ultrasounds and/or shock waves.

It is another object of the invention to provide an apparatus with an applicator handpiece which is resistant, light, ergonomic and easy to manage.

It is another object of the invention to provide an apparatus with an applicator handpiece which is automatic, high-quality, reliable, non-invasive, with low energy consumption and safe both for the user and the patient.

It is another object of the invention to provide an apparatus with an applicator handpiece which is obtainable in a simple and quick manner and at low costs.

All of these objects, both individually and in any combination thereof, and others, which will become apparent from the following description, are achieved, according to the invention, by an apparatus with an applicator handpiece having the features described in claim 1.

The present invention is hereinafter further clarified in a preferred practical embodiment thereof given by way of a mere non-limiting example with reference to the accompanying drawings, in which:

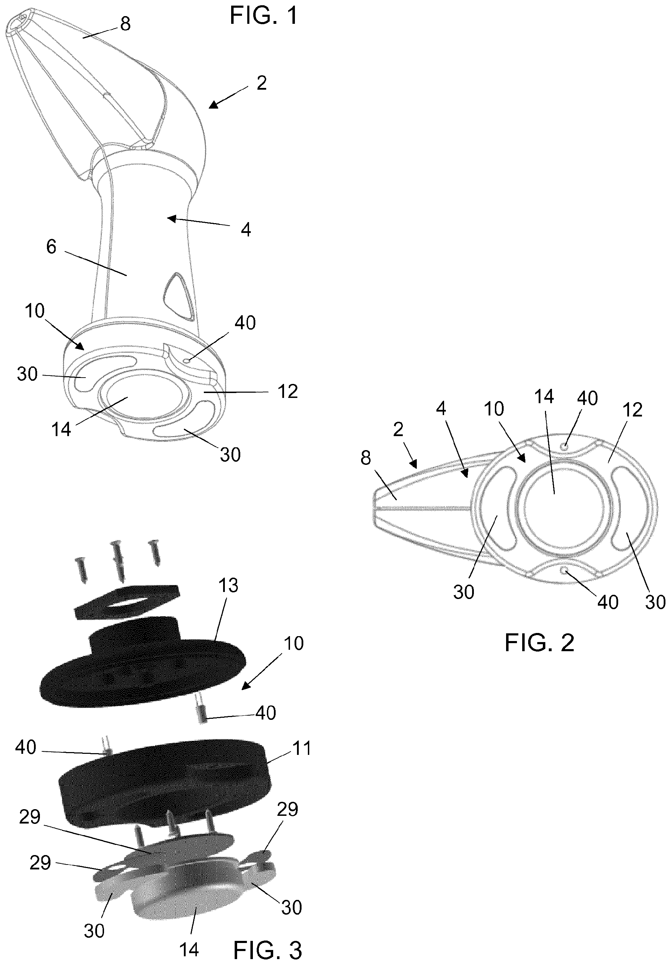

- Figure 1: shows in perspective bottom view of the applicator handpiece
- Figure 2: shows it in a plan bottom view,
- Figure 3: shows an exploded perspective view of a component thereof,
- Figure 4: shows the applicator handpiece in side view without a half-shell and schematically connected to a current/voltage generator,
- Figure 5: shows the wave shape of the electrical signal for the generation of the sound waves.

As shown in the figures, the improved applicator handpiece 2 comprises a body 4 which is provided with a grip 6 to allow the user to grasp and manage by hand the handpiece itself. In particular, the grip 6 comprises a central portion of the body 4 which is substantially cylindrical or slightly truncated-cone shaped.

Suitably, the body 4 has a shape globally similar to a gun. Advantageously, the body 4 comprises an upper portion 8, preferably "beak" shaped, which extends laterally from the grip 6.

Advantageously, the body 4 is formed by two mirror half-shells, suitably shaped, which are joined and kept together by traditional attachment means and/or by mechanical shape coupling.

Advantageously, the body 4 is made of a biocompatible polymer material with high mechanical and electrical insulation features. Preferably, the body 4 is made of acetal resin (POM-c), which is a semicrystalline copolymer with excellent features in terms of workability, stability, electrical insulation and, moreover, is physiologically inert.

Preferably, the grip 6 is covered with an anti-vibration rubber or a similar material, so as to not transmit any vibration to the hand of the user grasping said handpiece 2.

The applicator handpiece 2 also comprises a head 10 which is associated with the body 4 at the bottom. In particular, the lower surface 12 of the head 10 defines the portion of the applicator handpiece 2 intended to enter in direct contact with the skin or the body of the patient. Preferably, the lower surface 12 of the head 10 is substantially flat.

Advantageously, the head 10 comprises a support body 11 and a jointing element 13 made of biocompatible polymer material having high mechanical and electrical insulation features, such as for example acetal resin (POM-c).

At least one piezoelectric transducer 14 is housed inside the head 10, configured to transform the electrical energy supplied thereto into vibrational mechanical energy.

In particular, said at least one piezoelectric transducer 14 receives a first pulsed electrical signal configured to make said piezoelectric transducer generate shock waves to be directed on said zone to be treated, pressure - which is measured in output, i.e. at the zone to be treated - substantially comprised between 0.10 - 0.15 MPa (1 - 1.5 bars), and preferably of about 0.1 MPa (1 bar). Suitably, the piezoelectric transducer 14 only generates sound waves of pressure of about 0.1 MPa (1 bar) and in any case always and only less than 0.15 MPa (1.5 bars).

Suitably, the transducer 14 comprises at least one piezoelectric crystal, preferably only one, which is contained inside the body 25 of biocompatible metal material, preferably of medical steel alloy with high corrosion resistance (for example AISI 316). Preferably, the metal containment body 25 has a cylindrical shape with circular base having a diameter of about 40 mm and height of about 12 mm.

Preferably, the piezoelectric crystal is made of ceramic and this is particularly advantageous since, not oxidizing in contact with liquids or gels, it allows to lengthen the duration and useful lifespan thereof. Furthermore, the ceramic piezoelectric crystal is particularly resistant to mechanical fatigue and more malleable/easy to work, in particular, not requiring particular jointing processes at or along the edges thereof, thus allows to optimize the portion of the surface of the head 10 occupied by said crystal and thus increase the efficiency of the transducer 14.

In particular, the piezoelectric crystal contracts/expands with a frequency corresponding to that of the energizing/stimulating electric signal so as to generate pressure waves which cause a strong vibration of the surface 27 of the metal body 25 which is in contact with the zone of the body to be treated. Suitably, such an approaching and/or contact surface 27 is flat or slightly concave, or at most truncated cone shaped in order to better focus the vibration (beat) of the sound wave. Advantageously, the piezoelectric transducer 14 is configured so as to generate vibrations of amplitude substantially of 0.10 - 0.15 MPa (1 - 1.5 bars), preferably of about 0.1 MPa (1 bar), also with only one piezoelectric crystal and this allows to avoid the association of the transducer itself with a chamber containing liquid.

Furthermore, the piezoelectric transducer 14 is associated with an electrical transmission cable 16 which transports the electrical energy produced by a suitable current and/or voltage generator 18 to the transducer itself, which is outside the body 4 of the applicator handpiece 2 and may be main current and/or battery powered. In particular, the positive and negative poles of the electrical transmission cable 16 are connected to the upper surface (i.e. that opposite with respect to the surface 27 intended to come into contact with the zone of the body of the patient to be treated) of the metal containment body 25 of the piezoelectric transducer 14.

In particular, as said, based on the electrical signal received by the generator 18, the transducer 14 generates corresponding shock waves, i.e. vibrational mechanical waves (pressure waves). More in detail, the sequence and dispensing method of the mechanical pressure waves on the tissue follow the pattern of electrical signals which the generator 18 sends to the transducer 14.

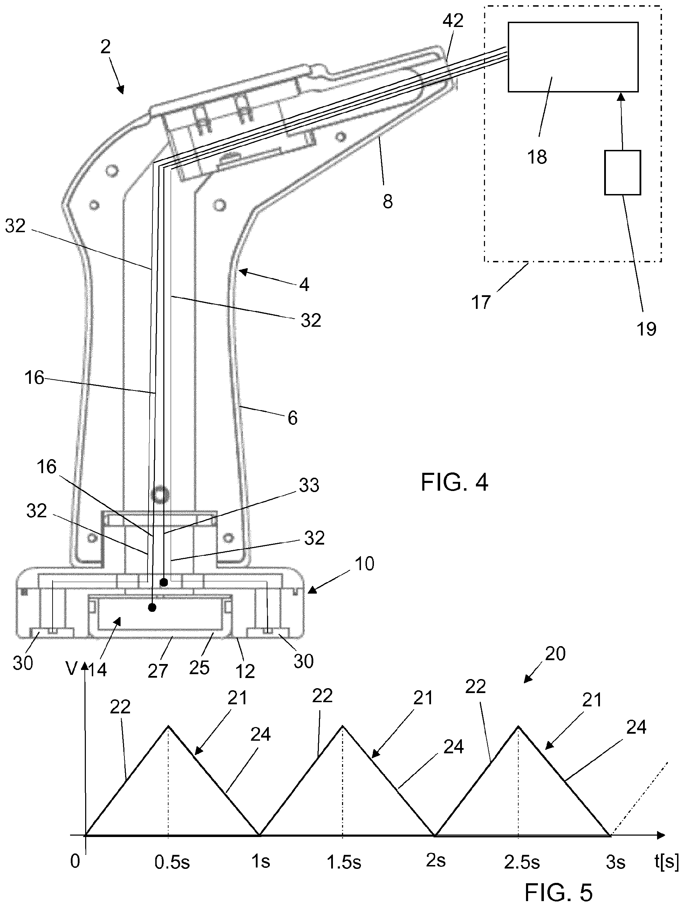

Suitably, in order to generate the shock waves, an electrical signal composed of a train of pulses 20 (see fig. 5), wherein each pulse 21 has a rise time 22 of about 0.5 s and a fall time 24 of about 0.5 s, is sent to the piezoelectric transducer 14. Preferably, the period of each pulse 21 is about 1 s, with a duty cycle of about 50%.

Preferably, the piezoelectric transducer 14 is stimulated with suitable electrical signals so as to generate shock waves with frequency of about 1-3 MHz and output power of about 3-9 W/cm²; suitably, the shock waves with these frequency and power values constitute an optimal selection for use both in the beauty field and in the medical field.

The upper head 10 also houses at least two shaped plates 30, which suitably act as electrodes, to transfer a diathermic current to the tissues to be treated. In particular, diathermic current means an electric current at high frequency, i.e. of frequency equal to or greater than about 480 kHz.

In particular, the pair of shaped plates 30 receives a second electrical signal of frequency equal to or greater than 480 kHz to thus generate diathermic currents to be transferred to said zone to be treated.

Advantageously, electrical signals are sent to the shaped plates 30 with carrier frequency equal or greater than about 480 kHz and with modulating frequency substantially comprised between 1 and 10 Hz, preferably equal to about 10 Hz, and with duty cycle preferably of about 90%, suitably, such electrical signals with these carrier and modulating frequencies constitute an optimal selection to obtain at the same time both the heating of the zone to be treated, which is caused by the diathermic currents generated by electrical carrier signals of frequency equal or greater than 480 kHz, and the transdermal transport - which is obtained by electrical modulating signals of frequency from 1 a 10 Hz, preferably of about 10 Hz - of the phytochemical, preferably pharmaceutical, which is applied on the skin of the zone to be treated.

In particular, each shaped plate 30 comprises a portion of biocompatible metal material, preferably of medical steel alloy with high corrosion resistance (for example AISI 316), which is connected, by means of a corresponding electric power cable (32), to the current and/or voltage generator 18, or to another suitable generator, for transferring the diathermic current to the tissues to be treated. Preferably, the shaped plates 30 comprise two flat or slightly concave semi-arched portions.

Advantageously, lighting means 40, for example LED, which are positioned to allow the user to illuminate the zone of the tissue on which the applicator handpiece 2 operates, are also associated with the upper surface 12 of the head 10. In particular, corresponding power supply wires 33 are associated with the lighting means 40 to provide to the means themselves the electric power supply energy provided by the current and/or voltage generator 18, or by another suitable generator. Advantageously, the activation signal of the lighting means 40 is synchronized with the rise time 22 of the stimulating electrical signal 20 sent to the piezoelectric transducer 14.

Preferably, in the embodiment shown in the figures, the piezoelectric transducer 14 is positioned in the middle of the head 10, while the lighting means 40 and the shaped plates 30 for the diathermy are positioned about it and are in pairs facing along two orthogonal diameters.

Preferably, inside the head 10, the piezoelectric transducer 14 and the shaped plates 30 are associated with suitable dielectric layers 29 to avoid, in particular for electric safety reasons, the dispersion of electrical energy.

The transmission cable 16 of the energizing electrical signal 20 to the piezoelectric transducer 14, the electric power cables 32 for the plates for the diathermy 30 and the electric power supply wires 33 of the lighting means 40 may be contained inside a single containment tube-shaped sheath, not shown, which is housed in and crosses the entire body 4 of the handpiece 2. In more detail, the sheath with the respective cables 16, 32 and optionally with the wires 33, enters inside the body 4 at an opening 42 defined at the upper portion 8, and after having crossed the entire body 4, arrives at the head 10 wherein the corresponding cables or wires, preferably contained within the sheath, are suitably divided to be connected to the piezoelectric transducer 14, to the electrodes for the diathermy 30 and to the lighting means 40.

Once the cable connectors 16, 32 and 33, which come out of the applicator handpiece 2, or the connector associated with the containment sheath thereof, connected to the corresponding connector provided in the device 17 which houses the electrical voltage/current generator 18 with the control unit 19 thereof, the user grips the handpiece itself at the grip 6 so as to rest the upper surface 12 of the head 10 in contact with the zone of the body of the patient to be treated.

Therefore, once that the user has activated the generator and set the dispensing profile more suitable for the specific application, both in terms of power (for example at 3, 6 or 9 W) and of number of beats, the tissues, which are in range of the handpiece 2, are affected by the shock waves generated by the energization of the piezoelectric transducer 14 and by the diathermic currents which come out of the shaped plates 30.

Advantageously, the heating of the tissues caused by the diathermic currents which come out from the shaped plates 30 makes the beats (vibrations) of the shock waves generated by the piezoelectric transducer 14 less painful.

Suitably, before or during the treatment with the handpiece, at the upper surface 12 of the head 10 thereof, or directly on the skin of the zone to be treated, a suitable phytochemical is applied, preferably pharmaceutical, in the form of liquid or gel, to help the transdermal transfer. In particular, the phytochemical facilitates the sliding of the handpiece on the skin reducing the natural electrical resistance thereof, and thus allows the phytochemical fluids thereof to penetrate inside the skin without injection.

Advantageously, the heating caused by the diathermic currents which come out of the shaped plates 30 favors the phytochemical transfer, preferably of a pharmaceutical, as the consequent increase in temperature of the tissues causes a weakening of the tissue ligaments, thus allowing a greater absorption of the substances.

Based on the foregoing, it is apparent that the apparatus with an applicator handpiece according to the invention is more advantageous with respect to those traditional since:
- simultaneously dispensing shock waves and diathermic currents allows to improve the efficiency and optimize the overall therapeutic and/or cosmetic treatment,
- it provides the user/therapist with a single instrument by means of which it is possible to intervene on a large number of pathologies in which the treatment by diathermy and/or shock waves is clinically validated,
- it is suitable for the application in dermatology, physiatrics, rheumatology, pain therapy, sport medicine, etc.,
- it is safe, biocompatible, easy and intuitive to use and manage for the user, in particular, the fact that the grip is mechanically distinct and separate from the lower base, which is intended to vibrate following the stimulation of the piezoelectric transducer, avoids or at least reduces the transmission of mechanical vibrations to the hand of the user,
- due to the constructional simplicity thereof it allows a saving of production costs.

In more detail, the apparatus with an applicator handpiece according to the invention allows to carry out a therapeutic and/or cosmetic treatment which synergistically combines diathermy and shock waves. In fact, the action of the diathermic currents generates a thermal effect, i.e. an increase of temperature, which weakens the electrochemical ligaments existing inside the tissue, so that the action of the pressure wave generated by the piezoelectric transducer then completely breaks these ligaments, thus allowing to form new ones through cell regeneration.

In particular, in contrast to WO2013/076716, EP2614807, WO2007/093998 and US2011/0015549, the present invention does not provide a generic ultrasound transducer, but provides a generator of shock waves, which are mechanical waves with wave shapes characterized by a quick peak (pulse) of positive pressure followed by a peak of negative pressure and capable of producing a direct mechanical stimulation.

Furthermore, in contrast to DE102011102570, US2010/0137752 and WO2014/138582, the present invention provides, besides a transducer for the generation of shock waves which is specifically of piezoelectric type, electrodes which receive electrical signals at high frequency (i.e. equal or greater than 480 kHz) for the generation of diathermic currents.

In substance, in contrast to the present invention, none of the apparatuses with applicator handpieces of the known type has the specific combination of a piezoelectric transducer configured for the generation of shock waves and of a pair of plates (electrodes) configured to generate diathermic currents to transfer to the zone to be treated. In particular, through the diathermic currents generated by the electrodes, tissue modifications are caused such as to allow the shock wave of a pressure of 0.10 - 0.15 MPa (1 - 1.5 bars) preferably of about 0.1 MPa (1 bar), generated by the piezoelectric transducer, to efficiently extend in the zone to be treated, thus avoiding the need to use much higher (i.e. from: 0.2 up to 0.5 MPa, i.e. from 2 up to 5 bars) pressure shock waves - as provided in traditional solutions, in particular of the pneumatic type - which are particularly painful for the patient. In other words, the use of diathermy along with the application of the shock waves allows to reduce the pressure of the shock waves to values of 0.10 - 0.15 MPa (1 - 1.5 bars), preferably of around 0.1 MPa (1 bar) while obtaining the same effectiveness of the treatment.

## Claims

1. An apparatus for therapeutic and/or cosmetic treatments, comprising at least one current and/or voltage generator (18) with a control unit (19) and an applicator handpiece (2) having a head (10) configured to be placed in contact with a zone to be treated of a body of a subject to be treated,
wherein said at least one current and/or voltage generator (18) is controlled by said control unit (19) to send a first pulsed electrical signal (20) and at least a second electrical signal,
wherein said head (10) comprises:
- at least one piezoelectric transducer (14) configured to receive said first pulsed electrical signal (20) being configured to make said piezoelectric transducer generate shock waves of a pressure of 0.10 - 0.15 MPa (1 - 1.5 bars) to be directed on said zone to be treated, and
- at least one pair of shaped plates (30) configured to receive said at least a second electrical signal of frequency equal to or greater than 480 kHz to thus generate diathermic currents to be transferred to said zone to be treated. and wherein said first pulsed electrical signal for said piezoelectric transducer (14) comprises a train of pulses (20) in which each pulse (21) has a rise time (22) of about 0.5 s and a fall time (24) of about 0.5 s.

2. An apparatus according to claim 1, wherein said handpiece (2) further comprises a body (4) provided with a grip (6) and in that said head is associated with one end of said body (4).

3. An apparatus according to claim 2, wherein said body (4) comprises a portion (8) which extends laterally at one end of said grip (6) and in that said head (10) is associated with the base of the other end of said grip (6).

4. An apparatus according to claims 2 or 3, wherein said body (4) is made of a biocompatible polymer material having high mechanical and electrical insulation features.

5. An apparatus according to one or more of the preceding claims, wherein said piezoelectric transducer (14) comprises at least one piezoelectric crystal, preferably of ceramic material, housed inside of a containment body (25) which in turn is housed in said head (10), said containment body (25) being made of biocompatible metal material with high corrosion resistance.

6. An apparatus according to claim 5, wherein said at least one piezoelectric crystal contracts/expands with a frequency corresponding to that of said first pulsed electrical signal (20) which is sent by first electrical transmission means (16), so as to generate corresponding pressure pulses on a surface (27) of the metal containment body (25) which is intended to be in contact with the zone to be treated.

7. An apparatus according to one or more of the preceding claims, wherein said piezoelectric transducer (14) is configured so as to generate shock waves of a pressure of 0.10 - 0.15 MPa (1 - 1.5 bars), with frequency greater than 1 MHz and output power of 3-9 W/cm².

8. An apparatus according to one or more of the preceding claims, wherein said at least a second electrical signal sent to the at least one pair of shaped plates (30) comprises an electrical carrier signal of frequency equal to or greater than about 480 kHz and a modulating electrical signal of frequency from 1 to 10 Hz, preferably of about 10 Hz.

9. An apparatus according to one or more of the preceding claims, wherein each shaped plate (30) comprises a semi-arched flat portion made of biocompatible material with high corrosion resistance.

10. An apparatus according to one or more of the preceding claims, wherein said head (10) also comprises lighting means (40) to illuminate said zone to be treated.

11. An apparatus according to claim 10, wherein said piezoelectric transducer (14) is positioned in the middle of a surface (12) of said head (10) intended to come into contact with the zone to be treated, said lighting means (40) and said shaped plates (30) for the diathermy are positioned at said surface (12) about said piezoelectric transducer and are in pairs facing along two orthogonal diameters.

12. An apparatus according to claims 10 or 11, wherein said lighting means (40) are configured to be activated by a third electrical signal which is synchronized with the rise time (22) of said first pulsed electrical signal (20) sent to said piezoelectric transducer (14).

13. An apparatus according to one or more of claims 10-12 when dependent on claim 3, wherein a transmission means (16) of said first pulsed electrical signal (20) to said piezoelectric transducer (14) and a transmission means (32) of said second electrical signal to said shaped plates (30) for diathermic currents, and any supply means (33) of said lighting means (40), are housed in a single sheath which enters in said body at an opening (42) defined in said portion (8) which extends laterally, and then internally through said body (4) until reaching said head (10).

## Patentansprüche

1. Gerät für therapeutische und/oder kosmetische Behandlungen, umfassend mindestens einen Strom- und/oder Spannungsgenerator (18) mit einer Steuereinheit (19) und einem Applikatorhandstück (2), das einen Kopf (10) aufweist, der ausgelegt ist, um in Kontakt mit einer zu behandelnden Zone eines Körpers einer zu behandelnden Person angeordnet zu werden, wobei der mindestens eine Strom- und/oder Spannungsgenerator (18) von der Steuereinheit (19) gesteuert wird, um ein erstes gepulstes elektrisches Signal (20) und mindestens ein zweites elektrisches Signal zu senden, wobei der Kopf (10) umfasst:
- mindestens einen piezoelektrischen Wandler (14), der ausgelegt ist, um das erste gepulste elektrische Signal (20) zu empfangen, das ausgelegt ist, um den piezoelektrischen Wandler zum Erzeugen von Stoßwellen mit einem Druck von 0,10-0,15 MPa (1-1,5 bar) zu bringen, die auf die zu behandelnde Zone gerichtet werden sollen, und
- mindestens ein Paar geformte Platten (30), die ausgelegt sind, um das mindestens eine zweite elektrische Signal mit einer Frequenz von gleich oder größer als 480 kHz zu empfangen, um somit diathermische Ströme zu erzeugen, die auf die zu behandelnde Zone übertragen werden sollen,
und wobei das erste gepulste elektrische Signal für den piezoelektrischen Wandler (14) eine Abfolge von Impulsen (20) umfasst, in der jeder Impuls (21) eine Anstiegszeit (22) von etwa 0,5 s und eine Abfallszeit (24) von etwa 0,5 s aufweist.

2. Gerät nach Anspruch 1, wobei das Handstück (2) ferner einen Körper (4) umfasst, der mit einem Griff (6) versehen ist, und wobei der Kopf mit einem Ende des Körpers (4) verbunden ist.

3. Gerät nach Anspruch 2, wobei der Körper (4) einen Abschnitt (8) umfasst, der sich seitlich an einem Ende des Griffs (6) erstreckt, und wobei der Kopf (10) mit der Basis des anderen Endes des Griffs (6) verbunden ist.

4. Gerät nach Anspruch 2 oder 3, wobei der Körper (4) aus einem biokompatiblen Polymermaterial besteht, das hohe mechanische und elektrische Isolationsmerkmale aufweist.

5. Gerät nach einem oder mehreren der vorhergehenden Ansprüche, wobei der piezoelektrische Wandler (14) mindestens einen piezoelektrischen Kristall, vorzugsweise aus keramischem Material, umfasst, der im Inneren eines Aufnahmekörpers (25) untergebracht ist, der wiederum in dem Kopf (10) untergebracht ist, wobei der Aufnahmekörper (25) aus einem biokompatiblen Metallmaterial mit hoher Korrosionsbeständigkeit besteht.

6. Gerät nach Anspruch 5, wobei sich der mindestens eine piezoelektrische Kristall mit einer Frequenz zusammenzieht/ausdehnt, die der des ersten gepulsten elektrischen Signals (20) entspricht, das von dem ersten elektrischen Übertragungsmittel (16) gesendet wird, um entsprechende Druckimpulse auf einer Oberfläche (27) des Metallaufnahmekörpers (25) zu erzeugen, der dazu bestimmt ist, mit der zu behandelnden Zone in Kontakt zu stehen.

7. Gerät nach einem oder mehreren der vorhergehenden Ansprüche, wobei der piezoelektrische Wandler (14) ausgelegt ist, um Stoßwellen mit einem Druck von 0,10-0,15 MPa (1-1,5 bar), mit einer Frequenz über 1 MHz und einer Ausgangsleistung von 3-9 W/cm² zu erzeugen.

8. Gerät nach einem oder mehreren der vorhergehenden Ansprüche, wobei das mindestens eine zweite elektrische Signal, das an das mindestens eine Paar geformte Platten (30) gesendet wird, ein elektrisches Trägersignal mit einer Frequenz von gleich oder größer als etwa 480 kHz und ein modulierendes elektrisches Signal mit einer Frequenz von 1 bis 10 Hz, vorzugsweise von etwa 10 Hz, umfasst.

9. Gerät nach einem oder mehreren der vorhergehenden Ansprüche, wobei jede geformte Platte (30) einen halbbogenförmigen, ebenen Abschnitt umfasst, der aus einem biokompatiblen Material mit hoher Korrosionsbeständigkeit besteht.

10. Gerät nach einem oder mehreren der vorhergehenden Ansprüche, wobei der Kopf (10) auch Beleuchtungsmittel (40) umfasst, um die zu behandelnde Zone zu beleuchten.

11. Gerät nach Anspruch 10, wobei der piezoelektrische Wandler (14) in der Mitte einer Oberfläche (12) des Kopfes (10) angeordnet ist, die dazu bestimmt ist, mit der zu behandelnden Zone in Kontakt zu kommen, wobei die Beleuchtungsmittel (40) und die geformten Platten (30) für die Diathermie auf der Oberfläche (12) um den piezoelektrischen Wandler herum und in gegenüberliegenden Paaren entlang zwei orthogonalen Durchmessern angeordnet sind.

12. Gerät nach Anspruch 10 oder 11, wobei die Beleuchtungsmittel (40) ausgelegt sind, um durch ein drittes elektrisches Signal aktiviert zu werden, das mit der Anstiegszeit (22) des ersten gepulsten elektrischen Signals (20) synchronisiert ist, das an den piezoelektrischen Wandler (14) gesendet wird.

13. Gerät nach einem oder mehreren der Ansprüche 10-12, wenn sie von Anspruch 3 abhängen, wobei ein Übertragungsmittel (16) des ersten gepulsten elektrischen Signals (20) zu dem piezoelektrischen Wandler (14) und ein Übertragungsmittel (32) des zweiten gepulsten elektrischen Signals zu den geformten Platten (30) für diathermische Ströme und ein Versorgungsmittel (33) der Beleuchtungsmittel (4) in einer einzelnen Hülle untergebracht sind, die an einer in dem sich seitlich erstreckenden Abschnitt (8) definierten Öffnung (42) in den Körper und dann im Inneren durch den Körper (4) hindurch bis zum Erreichen des Kopfes (10) eingesetzt ist.

## Revendications

1. Appareil pour traitements thérapeutiques et/ou cosmétiques, comprenant au moins un générateur de courant et/ou de tension (18) avec une unité de commande (19) et une pièce à main applicatrice (2) ayant une tête (10) configurée pour être placée en contact avec une zone à traiter d'un corps d'un sujet à traiter, dans lequel ledit au moins un générateur de courant et/ou de tension (18) est commandé par ladite unité de commande (19) pour envoyer un premier signal électrique pulsé (20) et au moins un deuxième signal électrique, dans lequel ladite tête (10) comprend:
- au moins un transducteur piézoélectrique (14) configuré pour recevoir ledit premier signal électrique pulsé (20) étant configuré pour amener ledit transducteur piézoélectrique à générer des ondes de choc d'une pression d'environ 0,1 à 0,15 MPa (1 à 1,5 bar) à diriger vers ladite zone à traiter, et
- au moins une paire de plaques profilées (30) configurées pour recevoir ledit au moins un deuxième signal électrique de fréquence égale ou supérieure à 480 kHz pour générer ainsi des courants diathermiques à transférer vers ladite zone à traiter,
et dans lequel ledit premier signal électrique pulsé par ledit transducteur piézoélectrique (14) comprend un train d'impulsions (20) dans lequel chaque impulsion (21) a un temps de montée (22) d'environ 0,5 s et un temps de descente (24) d'environ 0,5. s.

2. Appareil selon la revendication 1, dans lequel ladite pièce à main (2) comprend en outre un corps (4) pourvu d'une poignée (6) et dans lequel ladite tête est associée à une extrémité dudit corps (4).

3. Appareil selon la revendication 2, dans lequel ledit corps (4) comprend une partie (8) qui s'étend latéralement à une extrémité de ladite poignée (6) et dans lequel ladite tête (10) est associée à la base de l'autre extrémité de ladite poignée (6).

4. Appareil selon les revendications 2 ou 3, dans lequel ledit corps (4) est fait d'un matériau polymère biocompatible ayant des caractéristiques d'isolation mécanique et électrique élevées.

5. Appareil selon une ou plusieurs des revendications précédentes, dans lequel ledit transducteur piézoélectrique (14) comprend au moins un cristal piézoélectrique, de préférence en matériau céramique, logé à l'intérieur d'un corps de confinement (25) qui à son tour est logé dans ladite tête. (10), ledit corps de confinement (25) étant fait d'un matériau métallique biocompatible avec une résistance élevée à la corrosion.

6. Appareil selon la revendication 5, dans lequel ledit au moins un cristal piézoélectrique se contracte/se dilate avec une fréquence correspondant à celle dudit premier signal électrique pulsé (20) qui est envoyé par des premiers moyens de transmission électrique (16), de manière à générer des impulsions de pression correspondantes sur une surface (27) du corps de confinement métallique (25) qui est destinée à être en contact avec la zone à traiter.

7. Appareil selon une ou plusieurs des revendications précédentes, dans lequel le transducteur piézoélectrique (14) est configuré de manière à générer des ondes de choc d'environ 0,10-0,15 MPa (1-1,5 bars), avec une fréquence supérieure à 1 MHz et une sortie puissance de 3-9 W/cm².

8. Appareil selon une ou plusieurs des revendications précédentes, dans lequel ledit au moins un deuxième signal électrique envoyé à au moins une paire de plaques profilées (30) comprend un signal de porteuse électrique de fréquence égale ou supérieure à environ 480 kHz et un signal électrique modulant de fréquence de 1 à 10 Hz, de préférence d'environ 10 Hz.

9. Appareil selon une ou plusieurs des revendications précédentes, dans lequel chaque plaque profilée (30) comprend une partie plate semi-arquée en matériau biocompatible à haute résistance à la corrosion.

10. Appareil selon une ou plusieurs des revendications précédentes, dans lequel ladite tête (10) comprend également des moyens d'éclairage (40) pour éclairer ladite zone à traiter.

11. Appareil selon la revendication 10, dans lequel ledit transducteur piézoélectrique (14) est positionné au milieu d'une surface (12) de ladite tête (10) destinée à venir en contact avec la zone à traiter, lesdits moyens d'éclairage (40 ) et lesdites plaques profilées (30) pour la diathermie sont positionnées au niveau de ladite surface (12) autour dudit transducteur piézoélectrique et sont par paires se faisant face le long de deux diamètres orthogonaux.

12. Appareil selon les revendications 10 ou 11, dans lequel lesdits moyens d'éclairage (40) sont configurés pour être activés par un troisième signal électrique qui est synchronisé avec le temps de montée (22) dudit premier signal électrique pulsé (20) envoyé audit signal. transducteur piézoélectrique (14).

13. Appareil selon une ou plusieurs des revendications 10 à 12 lorsqu'elles dépendent de la revendication 3, dans lequel les moyens de transmission (16) dudit premier signal électrique pulsé (20) vers ledit transducteur piézoélectrique (14) et un moyen de transmission (32) dudit deuxième signal électrique auxdites plaques profilées (30) pour les courants diathermiques, et tout moyen d'alimentation (33) dudit moyen d'éclairage (40), sont logés dans une seule gaine qui entre dans ledit corps par une ouverture (42) définie dans ladite partie (8) qui s'étend latéralement, puis intérieurement à travers ledit corps (4) jusqu'à atteindre ladite tête (10).
